# EUROPÄISCHE PATENTSCHRIFT

(11) **EP 3 536 205 B1**
(45) Veröffentlichungstag und Bekanntmachung des Hinweises auf die Patenterteilung: **02.06.2021**
(21) Anmeldenummer: 18160089.1
(22) Anmeldetag: 06.03.2018
(51) Int. Cl.: A47J 43/07, A47J 37/06

(54) **SPEISENZUBEREITUNGSGERÄT**
FOOD PREPARATION APPARATUS
APPAREIL DE PRÉPARATION DES ALIMENTS

(43) Veröffentlichungstag der Anmeldung: 11.09.2019
(73) Patentinhaber: Vorwerk & Co. Interholding GmbH, 42270 Wuppertal (DE)
(72) Erfinder: Frielinghaus, Robert, 44799 Bochum (DE); Koetz, Hendrik, 58300 Wetter (DE)
(74) Vertreter: Gille Hrabal Partnerschaftsgesellschaft mbB Patentanwälte

(56) Entgegenhaltungen:
- DE-U1-202008 003 977
- US-A1- 2014 320 858
- US-A1- 2015 293 067
- Brian G. Osborne: "Near-Infrared Spectroscopy in Food Analysis : Applications, Theory and Instrumentation" In: "Encyclopedia of Analytical Chemistry", 30. Oktober 2000 (2000-10-30), Chichester [u.a.] : Wiley,, US, XP055502124, ISBN: 978-0-471-97670-7 DOI: 10.1002/9780470027318.a1018, * Zusammenfassung * * Seite 4, Spalte 2, Zeile 19 - Zeile 24 * * Seite 5, Spalte 1, Zeile 6 - Zeile 9 * * Seite 5, Spalte 1, Zeile 11 - Zeile 14 * * Seite 5, Spalte 2, Zeile 24 - Zeile 33 * * Seite 11, Spalte 1, Zeile 17 - Zeile 23 *

## Beschreibung

Die Erfindung betrifft ein Speisenzubereitungsgerät mit einem Speisenzubereitungsraum sowie einem Heizelement zum Erhitzen einer Speise in dem Speisenzubereitungsraum und/oder einem Werkzeug zum Mischen und/oder Zerkleinern einer Speise in dem Speisenzubereitungsraum. Die Erfindung betrifft zudem ein Verfahren.

Bei der Zubereitung einer Speise sind die Qualität des Kochergebnisses und die resultierenden Nährwerte von den zugeführten Speisebestandteilen und Kochparametern abhängig. Daher kommt es üblicherweise mitunter zu beträchtlichen Schwankungen bei der erzielten Qualität des Kochergebnisses und den resultierenden Nährwerten.

Besonders in Privathaushalten ist ein zunehmendes Interesse an gesunder Ernährung, an qualitativ hochwertigen Speisen und an einer genauen Kenntnis der Nährwerte selbst zubereiteter Speisen zu beobachten.

Die Druckschrift US2014/320858A1 offenbart ein Spektrometer, das für den Einsatz in mobilen Geräten wie Mobiltelefonen geeignet ist und dadurch einem Endverbraucher die Durchführung von Vor-Ort-Bestimmungen der Lebensmittelqualität ermöglicht.

Die Druckschrift Brian G. Osborne "Near-Infrared Spectroscopy in Food Analysis: Applications, Theory and Instrumentation" in Encyclopedia of Analytical Chemistry, 30. Oktober 2000, Chichester, ISBN: 978-0-471-97670-7, DOI: 10.1002/9780470027318.a1018, offenbart Anwendungen der NIR-Spektroskopie zur Nahrungsmittelanalyse insbesondere bei der industriellen Nahrungsmittelherstellung.

Es wird ferner auf die Druckschriften US2015/293067 und DE202008003977U1 hingewiesen.

Es ist Aufgabe der Erfindung, ein weiterentwickeltes Speisenzubereitungsgerät nebst Verfahren bereitzustellen.

Zur Lösung der Aufgabe dienen ein Speisenzubereitungsgerät gemäß Anspruch 1 sowie ein Verfahren gemäß Anspruch 10. Vorteilhafte Ausführungsformen ergeben sich aus den Unteransprüchen.

Zur Lösung der Aufgabe dient ein Speisenzubereitungsgerät mit einem Speisenzubereitungsraum sowie einem Heizelement zum Erhitzen einer Speise in dem Speisenzubereitungsraum und/oder einem Werkzeug zum Mischen und/oder Zerkleinern einer Speise in dem Speisenzubereitungsraum. Erfindungsgemäß umfasst das Speisenzubereitungsgerät ein Spektrometer zum Analysieren einer Speise.

Ein Speisenzubereitungsgerät kann beispielsweise ein Ofen, ein Kochautomat oder eine elektrische Küchenmaschine insbesondere mit einem Werkzeug zum Mischen und/oder Zerkleinern sein, das optional auch erhitzen kann.

Speisenzubereitungsraum meint den Raum oder Behälter, dem eine Speise oder ein Speisebestandteil zugeführt werden und in dem die Speise zubereitet wird. Zubereiten einer Speise bedeutet ein Verarbeiten durch Mischen, Zerkleinern und/oder Erhitzen. Eine Speise kann fest oder flüssig sein. Auch ein zuzuführender Speisebestandteil kann für sich genommen eine Speise sein wie z.B. Milch oder Trinkwasser.

Ein Spektrometer ist eine insbesondere optoelektronische Einrichtung, die eine elektromagnetische Strahlung in ihr Spektrum zu zerlegen vermag, derart, dass die Intensität der Strahlung in Abhängigkeit von der Wellenlänge gemessen wird. Bevorzugt kann ein Spektrometer durch ein Spektroskop und mindestens einen Strahlungsdetektor gebildet werden. Ein Spektroskop ist eine optische Einrichtung, mit dem elektromagnetische Strahlung in sein Spektrum zerlegt werden kann, wobei die Strahlung in Abhängigkeit von der Wellenlänge unterschiedlich abgelenkt wird. Insbesondere weist das Spektroskop eine spektroskopische Aufspaltungseinrichtung auf, z.B. ein Gitter, Prisma oder Interferometer. Beispielsweise lässt sich so weißes Licht zerlegen und das Spektrum regenbogenartig auf einer Leinwand abbilden. Ein Strahlungsdetektor ist eine bevorzugt elektronische Einrichtung zum Messen der Intensität einer einfallenden Strahlung. Vorzugsweise ist oder umfasst der Strahlungsdetektor mindestens eine Photodiode, die bei einer einfallenden elektromagnetischen Strahlung ein zu dessen Intensität korrelierendes Messsignal abgibt. Alternativ oder ergänzend kann der Strahlungsdetektor ein CCD-Sensor sein.

Hauptnährstoffe, Speisebestandteile oder Zustände der Speise können bevorzugt über einen sogenannten spektralen Fingerabdruck identifiziert werden, also anhand einer speziellen wellenlängenabhängigen Intensitätscharakteristik, z.B. vordefinierte Peaks bei vordefinierten Wellenlängen. Bevorzugt kann somit durch einen Vergleich der Messsignale mit hinterlegten Referenzwerten eine Speise, Eigenschaft oder ein Zustand zugeordnet werden.

Bei einem Spektrometer, das aus einem Spektroskop und einem Strahlungsdetektor gebildet ist, können in einer Ausführungsform ein oder mehrere Strahlungsdetektoren an vordefinierten Stellen installiert werden, zu denen jeweils ein Anteil der Messstrahlung mit einer bestimmten Wellenlänge abgelenkt oder hingeleitet wird. Durch den beschriebenen Aufbau kann dadurch mit einem besonders einfach und kompakt aufgebauten Spektrometer eine Speise analysiert werden. Alternativ oder ergänzend ist das Spektrometer so eingerichtet, dass der Strahlungsdetektor entlang der wellenlängenabhängig abgelenkten Messstrahlung gefahren wird, damit in Abhängigkeit von der Position eine gemessene Intensität der entsprechenden Wellenlänge zugeordnet werden kann.

Durch ein Speisenzubereitungsgerät mit einem Spektrometer zum Analysieren einer Speise kann eine Speise oder ein Speisebestandteil identifiziert, deren Eigenschaft erfasst oder deren Zustand ermittelt werden und zwar vor, während oder nach der Zubereitung. Bislang waren solche Informationen einem Speisenzubereitungsgerät unbekannt. Durch den Einsatz eines Spektrometer können Informationen dieser Art dem Speisenzubereitungsgerät nun zugänglich gemacht werden und dadurch ein besonders reproduzierbares Kocherergebnis und eine besonders genaue Kenntnis der tatsächlich erhaltenen Nährwerte erreicht werden.

Beispielsweise kann auf Basis der tatsächlich zugeführten Speisebestandteile vor der Zubereitung und/oder dem tatsächlichen Zustand der Speise während der Zubereitung eine Prozessüberwachung und Optimierung des Zubereitungsprozesses durch ein Regeln einzelner Kochparameter wie Temperatur und Zeit oder durch ein Auffordern des Benutzers zur Zugabe eines bestimmten Speisebestandteils wie z.B. Zucker oder Wasser erfolgen. Nach der Zubereitung können Hauptnährstoffe, d.h. Kohlenhydrate, Fett und Eiweiß, ermittelt und der Energiegehalt der zubereiteten Speise in Kilokalorie (kcal) berechnet werden.

Der Einsatz einer großen Menge von Einzelsensoren jeweils zur Erfassung einer bestimmten Eigenschaft oder eines bestimmten Zustands wie z.B. Bräunungsgrad durch Kameratechnik, Alkoholkonzentrationen durch Refraktometern oder Wassergehalt durch differentielle Thermometrie kann auf diese Weise ebenso entfallen wie ein rezeptbasiertes Abschätzen der Nährstoffe und des Energiegehaltes mithilfe von Nährwert- und Kalorienübersichten. Ein besonders einfach aufgebautes und mit geringem Aufwand herstellbares Speisenzubereitungsgerät mit der oben beschriebenen Funktionalität kann so bereitgestellt werden. Darüber hinaus wird ein hoher Bedienkomfort bei geringem Bedienaufwand erzielt. Schließlich kann auch die Betriebssicherheit z.B. bei Destillation von Alkohol erhöht werden.

In einer Ausführungsform ist eine Strahlquelle für das Analysieren einer Speise vorgesehen. Eine Strahlquelle emittiert elektromagnetische Strahlung zum Einbringen von Energie in die zu analysierende Speise, auch Anregungsstrahlung genannt. Bevorzugt ist eine breitbandige Strahlquelle, z.B. ein Globar, eine Quarzhalogenlampe oder Hg-Hochdrucklampe, mit einem mittels Referenzmessung oder Kalibrierung bekannten Spektrum vorgesehen. Alternativ oder ergänzend wird ein Diodenlaser als Strahlquelle eingesetzt. Durch eine Strahlquelle kann eine besonders präzise Analyse erreicht werden.

Vorzugsweise emittiert die Strahlquelle Infrarot-Strahlung. Insbesondere umfasst Infrarot-Strahlung nahe Infrarotstrahlung (NIR) mit einem Wellenlängenbereich von 780 nm bis 1.400 nm, kurzwellige Infrarotstrahlung (SWIR) im Bereich von 1.400 nm bis 3.000 nm, mittlere Infrarotstrahlung (MWIR) im Bereich von 3.000 nm bis 8.000 nm, langwellige Infrarotstrahlung (LWIR) im Bereich von 8.000 nm bis 15.000 nm und/oder ferne Infrarotstrahlung (FIR) im Bereich von 15.000 nm bis 1.000.000 nm. Bevorzugt ist eine Strahlquelle vorgesehen, die Anregungsstrahlung im NIR-Bereich emittiert. Eine Speise oder Speisebestandteile können dadurch besonders zuverlässig analysiert werden.

Zur Analyse einer Speise trifft die Anregungsstrahlung, d.h. Photonen, der Strahlquelle auf die zu analysierende Speise. Dort werden die Moleküle durch diese eintreffende oder eingebrachte Energie angeregt. In Abhängigkeit von der Speise, dessen Eigenschaften oder Zustand wird die Anregungsstrahlung zu bestimmten Anteilen der eintreffenden Anregungsstrahlung durch die Speise reflektiert, absorbiert oder transmittiert. Insbesondere wird die Reflektionsstrahlung und/oder die Transmissionsstrahlung als die durch den Strahlungsdetektor zu erfassende Messstrahlung genutzt. Bevorzugt wird die Reflektionsstrahlung als die Messstrahlung genutzt. Mit Messstrahlung ist die für die Analyse der Speise zu analysierende Strahlung der Speise oder eines Speisebestandteils gemeint.

Erfindungsgemäß ist eine optische Faser vorgesehen, um eine Messstrahlung von einer Speise zu dem Spektrometer zu leiten und/oder eine Anregungsstrahlung von der Strahlquelle zu einer Speise zu leiten. Eine optische Faser vermag elektromagnetische Strahlung, insbesondere im NIR-Bereich, zu leiten. Die optische Faser, die bevorzugt aus Glas oder Kunststoff hergestellt ist, erlaubt ein Biegen. Durch den Einsatz einer optischen Faser können die Strahlquelle und/oder der Strahlungsdetektor somit an einer beliebigen Position im oder am Speisenzubereitungsgerät angeordnet oder installiert sein. Bevorzugt weist ein Hauptgerät die Strahlquelle und/oder den Strahlungsdetektor für eine kompakte Integration in das Speisenzubereitungsgerät auf. Alternativ oder ergänzend kann auch ein separates, jedoch mit dem Hauptgerät elektrisch verbindbares Gefäß mit dem Speisenzubereitungsraum die Strahlquelle und/oder den Strahlungsdetektor aufweisen.

In einer Ausführungsform verläuft die optische Faser, insbesondere der Strahlquelle und/oder des Strahlungsdetektors, durch eine Öffnung in einem Gefäß mit dem Speisenzubereitungsraum oder einem Deckel für den Speisenzubereitungsraum. Auf diese Weise kann die Speise innerhalb des Speisenzubereitungsraums analysiert werden, ohne die Speise selbst zu beeinträchtigen oder den Zubereitungsprozess zu stören.

Bevorzugt wird die bestehende mittige Öffnung eines Deckels zum Durchführen der mindestens einen optischen Faser genutzt. Insbesondere ist dann ein Verschluss für die Öffnung vorgesehen, um die Öffnung mit durchgeführter optischer Faser dicht verschließen zu können. Bevorzugt ist die Öffnung in einem Gefäß an einem oberen Rand, Randbereich oder oberen Bereich des Gefäßes angeordnet. Bevorzugt ist eine Durchgangsöffnung als die Öffnung vorgesehen, um die optische Faser von außen in den Speisenzubereitungsraum zu führen. Bevorzugt weist die Öffnung eine Querschnittsfläche auf, die der Querschnittsfläche der optischen Faser oder von zwei optischen Fasern optional zuzüglich der Querschnittsfläche einer Dichtung entspricht, um ein oder zwei optische Fasern platzsparend und dicht in den Speisenzubereitungsraum zu führen. Bevorzugt ist eine Dichtung vorgesehen, um die Öffnung mit der oder den optischen Fasern abzudichten. Insbesondere weist die Öffnung eine Neigung zur Außenoberfläche des Deckels bzw. des Gefäßes auf, um einen Zielbereich in dem Speisenzubereitungsraum zu analysieren. Vorzugsweise sind mehrere unterschiedlich ausgerichtete Öffnungen oder eine Schwenkeinrichtung vorgesehen, damit besonders flexibel der Zielbereich für die Analyse in dem Speisenzubereitungsraum verändert und eingestellt werden kann. Bevorzugt sind die optische Faser und die Öffnung so eingerichtet, dass ein manuelles Anbringen und Lösen der optischen Faser in der Öffnung bzw. von der Öffnung vorgesehen ist. Eine beschädigte optische Faser kann so durch den Benutzer einfach und schnell ausgetauscht werden. Ferner kann so die optische Faser nur dann eingesetzt werden, wenn eine Analyse gewünscht ist und andernfalls die Öffnung mit einem Verschluss geschlossen werden. Alternativ oder ergänzend kann die optische Faser derart fest mit der Öffnung verbunden sein, dass diese Verbindung nicht für ein Lösen durch den Benutzer vorgesehen ist.

In einer Ausführungsform verläuft die optische Faser, insbesondere der Strahlquelle und/oder des Strahlungsdetektors, durch eine Öffnung durch das Werkzeug. Dies hat den Vorteil, dass die optische Information direkt aus dem Innenraum des Speisenzubereitungsraums bezogen wird und nicht von außen erhalten wird. Oberflächeneffekte wie z.B. die Haut auf einem Pudding, die sonst das Analyseergebnis beinträchtigen würden, können auf diese Weise eliminiert werden. Bevorzugt verläuft die mindestens eine optische Faser durch das Werkzeug zum Mischen und/oder Zerkleinern entlang der Werkzeugachse zum Speisenzubereitungsraum. Zentrifugalkräfte auf die optische Faser können so vermieden werden. Insbesondere ist ein Drehlager und/oder eine Dichtung vorgesehen, um die optische Faser drehflexibel bzw. dicht durch das Werkzeug zu führen.

Insbesondere fluchtet die optische Faser mit einer Innenkontur des Speisenzubereitungsraums, d.h. sie steht nicht über eine Gefäßinnenwand oder eine Werkzeugaußenkontur hervor. Eine Beschädigung der Faser und Faserrückstände in der Speise können so besonders wirksam vermieden werden. Die Faser ist vorzugsweise aus bioverträglichem Material.

In einer Ausführungsform ist eine optische Kupplungsschnittstelle zum Anschließen der optischen Faser an das Spektrometer, die Strahlquelle, das Gefäß, den Deckel und/oder das Werkzeug vorgesehen oder jeweils eine optische Kupplungsschnittstelle dazu eingerichtet. Eine optische Kupplungsschnittstelle erlaubt ein Leiten einer Anregungsstrahlung oder Messstrahlung zwischen zwei optischen Einrichtungen, wie z.B. zwei optischen Fasern oder zwischen der optischen Faser und einer Optik zur Formung eines Strahlengangs. Eine Optik zur Formung eines Strahlengangs ist bevorzugt am Ausgang der Strahlquelle und am Eingang des Spektrometers vorgesehen. Insbesondere kann eine Optik in dem Gefäß, dem Deckel oder dem Werkzeug integriert sein, um die Speise und den Zubereitungsprozess besonders wenig zu beeinträchtigen und diesen Bedingungen mit besonders geringem Verschleiß standhalten zu können. Im einfachsten Fall kann eine Optik ein Analysefenster sein. Durch eine Optik kann ferner eine Bewegungseinrichtung oder eine Fokussiereinrichtung besonders einfach implementiert werden.

Eine optische Kupplungsschnittstelle an einem außerhalb des Speisenzubereitungsraums befindlichen Teil des Werkzeugs, insbesondere auf der Werkzeugdrehachse, ermöglicht ein Übertragen der Anregungsstrahlung und/oder Messstrahlung von der optischen Faser in eine Optik oder einen separaten Abschnitt der optischen Faser innerhalb des Werkzeugs, also entkoppelt von einer Rotation des Werkzeugs. Wenn das Werkzeug rotiert, dreht die Optik oder der separate Abschnitt der optischer Faser mit dem Werkzeug mit, wobei dennoch das Übertragen der Anregungsstrahlung und/oder Messstrahlung von der dazu feststehenden optischen Faser erfolgt. Die Optik oder der separate Abschnitt der optischen Faser sind bei dieser Ausgestaltung mit dem Werkzeug drehfest verbunden.

Erfindungsgemäß ist eine optische Weiche vorgesehen, die so beschaffen ist, dass die Anregungsstrahlung durch die optische Faser und eine zusätzliche optische Faser geleitet werden kann oder die Messstrahlung durch die optische Faser und eine zusätzliche optische Faser geleitet werden kann. Eine optische Weiche ist ein Y-Verteiler. Es kann somit entweder Messstrahlung von der optischen Faser und der zusätzlichen optischen Faser zu demselben Spektrometer geleitet werden oder Anregungsstrahlung von nur einer Strahlquelle durch die optische Faser und die zusätzliche optische Faser geleitet werden. Mit einer optischen Weiche kann ermöglicht werden, dass eine zusätzliche optische Faser zusätzlich zu der optischen Faser an die Strahlquelle bzw. den Strahlungsdetektor angeschlossen oder angekoppelt werden kann. Eine Speise kann so an zwei verschiedenen Stellen analysiert werden. Ferner kann eine Speise innerhalb und eine Speise oder ein Speisenbestandteil außerhalb des Speisenzubereitungsraums durch dieselbe Strahlquelle und/oder denselben Strahlungsdetektor analysiert werden.

In einer Ausführungsform weist ein Gefäß mit dem Speisenzubereitungsraum oder ein Deckel für den Speisenzubereitungsraum ein Analysefenster auf. Analysefenster meint ein Fenster zum Analysieren einer Speise innerhalb des Speisenzubereitungsraums. Insbesondere ist das Analysefenster überwiegend durchlässig für die Anregungsstrahlung und/oder eine für die Analyse vorgesehene Messstrahlung. Eine für die Analyse vorgesehene Messstrahlung meint Reflektionsstrahlung oder Transmissionsstrahlung der Speise innerhalb mindestens eines Wellenlängenbereiches, der zur Erkennung eines vordefinierten spektralen Fingerabdrucks in einer Speichereinheit hinterlegt ist. Ein Analysefenster ist bevorzugt in Form einer Glasplatte umgesetzt. Durch ein Analysefenster wird eine besonders hohe Robustheit des Analysesystems sowie ein Analysieren der Speise unterhalb der Oberfläche der Speise ermöglicht. Ein Analysefenster kann ergänzend oder in Verbindung mit einer optischen Faser eingesetzt werden. Ein Analysefenster kann auch als Alternative zu einer optischen Faser eingesetzt sein. Es kann somit auf eine Faser verzichtet werden.

In einer Ausführungsform sind eine Bewegungseinrichtung und/oder eine Fokussiereinrichtung vorgesehen, so dass eine Anregungsstrahlung flexibel auf eine Speise gerichtet werden kann und/oder eine Messstrahlung von einer Speise flexibel erfasst werden kann. Flexibel meint aus einer anderen Position, unter einem anderen Winkel oder mit einer anderen Fokuseinstellung oder Brennweite. Insbesondere vermag die Bewegungseinrichtung ein Spektrometer, eine Strahlquelle oder ein Ende einer optischen Faser zu bewegen oder zu neigen. Insbesondere umfasst die Fokussiereinrichtung ein Linsensystem und/oder ist mit dem Eingang eines Spektrometers, dem Ausgang einer Strahlquelle, einem Ende einer optischen Faser oder einer Analysescheibe verbunden, d.h. optisch angekoppelt. Durch eine Bewegungseinrichtung kann beispielsweise mittels eines Analysefensters und/oder ohne Einsatz einer optischen Faser an verschiedenen Stellen der Speise eine Analyse vorgenommen werden. Durch eine Fokussiereinrichtung kann ein unterschiedlich großer Bereich der Speise analysiert oder eine besonders präzise Analyse ermöglicht werden. Durch die Bewegungseinrichtung und/oder Fokussiereinrichtung wird ein ortsselektives Analysieren ermöglicht.

In einer Ausführungsform ist eine Kamera vorgesehen, um eine Speise digital abzubilden. Eine Kamera ist eine optoelektronische Apparatur insbesondere zur elektronischen Aufnahme eines statischen oder bewegten Objektes im sichtbaren Wellenlängenbereich der von dem Objekt ausgehenden elektromagnetischen Strahlung. Digital abbilden meint Umwandeln in digitale Bildwerte, beispielsweise Pixelwerte. Durch das Vorsehen einer Kamera wird ein optisches Clustering, d.h. eine Objektidentifikation, ermöglicht. Örtlich getrennte Speisekomponenten wie z.B. eine Kartoffel neben einem Stück Fleisch können so erkannt und diese Information in die Analyse mit einbezogen werden. Zum Teil können einzelne vordefinierte Speisekomponenten in einer Ausführungsform auch mithilfe der Kamera und durch einen Vergleich mit hinterlegten Referenzbildmodellen erkannt werden.

In einer Ausführungsform sind das Spektrometer und/oder die Strahlquelle in einem Ofen oder einem Gefäß mit dem Speisenzubereitungsraum, in einen Deckel für den Speisenzubereitungsraum und/oder in einem Grundgerät zur Aufnahme des Gefäßes integriert oder mechanisch damit verbunden. Integrieren meint fest installiert. Die Integration ermöglicht eine besonders kompakte Bauweise.

In einer Ausführungsform sind das Spektrometer und die Strahlquelle als gemeinsame Analyseeinheit vorgesehen und/oder an einen Ofen, an ein Gefäß mit dem Speisenzubereitungsraum, an einen Deckel für den Speisenzubereitungsraum und/oder an ein Grundgerät zur Aufnahme des Gefäßes ankoppelbar. Gemeinsame Analyseeinheit meint, dass das Spektrometer und die Strahlquelle permanent miteinander verbunden sind, also ein Lösen durch den Benutzer nicht vorgesehen ist. Durch das Vorsehen des Spektrometers und der Strahlquelle als gemeinsame Analyseeinheit kann durch nur eine Bewegungseinrichtung die Analyseeinheit besonders effizient bewegt werden. Ferner wird ein portabler Einsatz der Analyseeinheit unterstützt. Ankoppelbar meint, dass das Spektrometer, die Strahlquelle oder die Analyseeinheit dazu eingerichtet sind, durch den Benutzer manuell mechanisch angekoppelt und entkoppelt, also gelöst und entnommen, zu werden. In einer Ausführungsform kann das Spektrometer eigenständig entnommen und portabel eingesetzt werden. In einer weiteren Ausführungsform kann die Strahlquelle eigenständig entnommen und portabel eingesetzt werden. Der portable Einsatz hat den Vorteil, dass z.B. außerhalb des Speisenzubereitungsraums eine zubereitete Speise oder ein zuzuführender Speisebestandteil analysiert werden kann.

In einer Ausführungsform können das Spektrometer, die Strahlquelle oder die Analyseeinheit mit einem Ofen, Gefäß oder Grundgerät zur Aufnahme des Gefäßes mechanisch permanent verbunden sein, d.h., dass die mechanische Verbindung nicht für ein manuelles Lösen durch den Benutzer eingerichtet ist. Dies vereinfacht die Messsignalverbindung und Stromversorgung. Alternativ oder ergänzend ist die mechanische Verbindung zwar als permanente Verbindung eingerichtet, jedoch flexibel ausrichtbar, um beispielsweise sowohl eine Speise innerhalb als auch außerhalb des Speisenzubereitungsraums analysieren zu können. Insbesondere erfolgt das mechanische Verbunden oder Ankoppeln in einem oberen Bereich oder einer Oberkante eines Gefäßes. Ein in den Deckel oder einen Deckelzwischeneinsatz integriertes Spektrometer und/oder Strahlquelle können in einer Ausführungsform reibschlüssig oder formschlüssig mit einer Oberkante eines Gefäßes verbunden werden.

In einer Ausführungsform ist das Speisenzubereitungsgerät so eingerichtet, dass vor der Zubereitung einer Speise ein Speisebestandteil für die zuzubereitende Speise analysiert wird oder die Speise in dem Zustand vor der Zubereitung analysiert wird. Vor der Zubereitung meint vor einem Mischen, Zerkleinern und/oder Erhitzen in dem Speisenzubereitungsraum. Ein Optimieren des Kochprozesses anhand der Analyse der Speise oder Speisebestandteile vor der Zubereitung sowie ein reproduzierbares Kochergebnis können so erzielt werden.

Insbesondere werden vor der Zubereitung ein Speisebestandteil außerhalb des Speisenzubereitungsraums und/oder noch zuzuführender Speisebestandteil außerhalb des Speisenzubereitungsraums analysiert. Dadurch kann eine besonders genaue Kenntnis der tatsächlich verwendeten Zutaten bzw. zuzuführenden Speisebestandteile und damit der Nährwert einer selbst zubereiteten Speise z.B. für das Einhalten einer Diät erlangt werden. Bislang können Nährwertangaben in Rezepten für selbst gekochte Speisen nur grob anhand generischer Daten der Zutaten oder Untersuchungen aus der Erprobungsphase des Rezeptes abgeschätzt werden. Eine Variation oder gar Substitution der eingefüllten Zutaten, d.h. der zuzuführenden Speisebestanteile, konnten bislang nicht abgebildet werden. Ebenfalls erfolgt bislang keine Berücksichtigung der tatsächlich verwendeten Zutaten.

Insbesondere werden vor der Zubereitung die Speise oder Speisebestandteile in dem Speisenzubereitungsraum analysiert. Der Kochprozess kann dadurch an schwankende Quantität oder Qualität des Ausgangszustands der Speise vor der Zubereitung bzw. der eingeführten Speisebestandteile angepasst werden, um selbst bei veränderten Mischungsverhältnissen, Rezeptveränderungen oder ohne Rezept zubereiteten Speisen ein reproduzierbares oder zumindest optimiertes Kochergebnis zu erhalten.

In einer Ausführungsform ist das Speisenzubereitungsgerät so eingerichtet, dass während der Zubereitung einer Speise die Speise oder ein Speisebestandteil der Speise analysiert wird. Die Analyse erfolgt innerhalb des Speisenzubereitungsraums. Eine dynamische Anpassung und/oder Regelung der Zubereitungsparameter wie z.B. Temperatur, Garzeit oder Drehzahl des Werkzeugs können so in Abhängigkeit von den identifizierten Speisebestandteilen, den aktuell vorliegenden Inhaltsstoffen und des Ist-Zustandes der Speise erfolgen. Ferner kann eine dynamische Anpassung der Zutatenliste während der Zubereitung bei Erkennung eines Mangels oder Überschusses eines speziellen Inhaltsstoffes vorgenommen und dem Benutzer angezeigt werden. Ein besonders optimiertes und/oder reproduzierbares Kochergebnis trotz schwankender Qualität und Quantität der anfangs zugeführten Speisebestandteile, also Nahrungsmittel bzw. Zutaten, sowie Veränderungen der Speisebestanteile während der Zubereitung beispielsweise durch Verdunstung oder Fermentierung kann so ermöglicht werden. Eine zerstörende oder speziell invasive Analyse und ein dadurch bedingtes Stören des Zubereitungsprozesses durch das Analysieren selbst kann ebenfalls vermieden werden.

Ist der Zielwert einer vordefinierten Überwachungsgröße erreicht, kann das Speisenzubereitungsgerät entsprechend vordefinierte Maßnahmen ergreifen, die in einer Speichereinheit gespeichert sind. Beispielhaft kann beim Garen oder Backen der Bräunungsgrad die Überwachungsgröße und der gewünschte Bräunungsgrad der Zielwert sein, so dass ein Heizelement bei Erreichen des Zielwerts abgeschaltet oder heruntergeregelt wird. Wenn der gewünschte Bräunungsgrad noch nicht erreicht ist, die gewünschte Kerntemperatur der Speise, also in diesem Fall des Garguts, jedoch bereits erreicht ist, kann beispielsweise ein Ofen als das Speisenzubereitungsgerät im Grillmodus betrieben werden, um rasch eine äußere Bräunung zu erreichen. Wenn der gewünschte Bräunungsgrad erreicht ist, die gewünschte Kerntemperatur des Speise aber noch nicht, kann die Temperatur heruntergeregelt oder der Nutzer aufgefordert werden, die Speise abzudecken.

In einer Ausführungsform sind speisenspezifische oder benutzerspezifische Stopp- oder Abbruchkriterien definiert. Dadurch kann beispielsweise ein Garprozess angehalten werden, wenn durch die Analyse ein Erreichen eines vorbestimmten Bräunungsgrades ermittelt wurde. Ferner kann dadurch in einer weiteren Ausführungsform eine Sicherheitsfunktion implementiert werden, die bei Erreichen einer vorbestimmten Alkoholkonzentration beispielsweise bei der Destillation von Alkohol in einer Küchenmaschine oder einem anderen Küchengerät den Zubereitungsprozess abbricht.

In einer Ausführungsform ist das Speisenzubereitungsgerät so eingerichtet, dass nach der Zubereitung einer Speise die Speise analysiert wird. Der Benutzer erhält dadurch eine genaue Information über den tatsächlichen Nährwert der zubereiteten Speise unabhängig von Rezeptabweichungen oder Eigenkompositionen. Eine schnelle, zerstörungsfreie und unkomplizierte Nährwertanalyse eines selbst zubereiteten Gerichts kann realisiert werden. Dem Benutzer wird es durch Analyse des Ist-Zustands der Speise vor dem Verzehr ferner ermöglicht, einen Diätplan besonders zuverlässig einhalten zu können. Ein Abschätzen, Abwiegen und ein händisches Ausrechnung mittels Nährwerttabellen kann komplett entfallen. In einer Ausführungsform erfolgt eine Verknüpfung mit einer Küchenmaschine zur Optimierung hinterlegter Kochparameter für eine spezielle Speise oder für eine Berücksichtigung des benutzen und durch die Küchenmaschine bereitgestellten Rezeptes für die Nährwertanalyse zum Erzielen einer besonders hohen Präzision des Analyseergebnisses. Wenn in einer Ausführungsform der Diätplan in einer Steuerungseinrichtung hinterlegt ist, kann ein Soll-Ist-Abgleich erfolgen und z.B. durch eine Küchenmaschine angezeigt werden. Bevorzugt wird die Speise innerhalb des Speisenzubereitungsraums analysiert, um die Analyse nach der Zuleitung besonders einfach voll automatisiert umsetzten zu können. Alternativ oder ergänzend ist auch ein Analysieren der Speise außerhalb des Speisenzubereitungsraums möglich.

In einer Ausführungsform ist eine Steuerungseinrichtung mit einer Prozessoreinheit und einer Speichereinheit vorgesehen. Die Steuerungseinrichtung ist insbesondere so beschaffen, dass mithilfe der Prozessoreinheit ein Verfahren ausgeführt werden kann, bevorzugt anhand eines in der Speichereinheit gespeicherten Computerprogramms oder Befehlen. Die hinterlegten Daten, d.h. mindestens ein Datensatz, Referenzwert und/oder Referenzbildmodell, sind vorzugsweise in der Speichereinheit hinterlegt, also digital gespeichert. Durch die Steuerungseinrichtung können die insbesondere analogen und/oder digitalen Messsignale des Spektrometers und optional einer Kamera verarbeitet werden. Bevorzugt wird bzw. werden eine Signalvorverarbeitung und/oder Signalkorrektur z.B. zum Kompensieren eines Temperatureinflusses oder zur Analog-Digital-Umwandlung durchgeführt, bevor die so erhaltenen vorverarbeiteten Messwerte mithilfe der hinterlegten Daten insbesondere unter Anwendung vordefinierter Algorithmen analysiert werden. Da während des Zubereitungsprozesses häufig erhöhte Temperaturen herrschen, die sich ebenfalls in einem Infrarotstrahlungsanteil widerspiegeln, ist in einer Ausführungsform eine Temperaturmessung vorgesehen, die bevorzugt durch einen bestehenden Temperatursensor z.B. einer Küchenmaschine oder eines Ofens erfolgt. Der Einfluss der Temperatur kann dann z.B. über ihre Planck-Kurve herausgerechnet werden. Insbesondere ist die Steuerungseinrichtung in dem Hauptgerät integriert. Ein vollständiges oder teilweises Auslagern der Steuerungseinrichtung und/oder der Speichereinheit mit den Datensätzen, Referenzwerten und/oder Referenzbildmodellen in eine Cloud sind in einer alternativen oder ergänzenden Ausführungsform vorgesehen. Die Cloud ist insbesondere mit einem Grundgerät des Speisezubereitungsgeräts für ein Austauschen von Daten verbunden. "In eine Cloud" meint ein Speichern von Daten und/oder ein Verarbeiten von Daten durch einen entfernten Computer, der beispielsweise über das Internet erreichbar ist. Dieser Computer kann in einer Ausführungsform Steuerbefehle zurücksenden.

In einer Ausführungsform ist ein spektraler Fingerabdruck in Form eines Datensatzes hinterlegt, der einer bestimmten Speise, einem bestimmten Speisebestandteil, einer bestimmten Eigenschaft oder einem bestimmten Zustand zugeordnet ist. Bevorzugt weist dieser Datensatz für eine oder mehrere vordefinierte Wellenlängen der Messstrahlung jeweils eine vordefinierte Intensitätsschwelle oder einen vordefinierten Intensitätsbereich auf. Zeigt ein Vergleich der Messstrahlung mit den Datensätzen, dass die Bedingungen eines bestimmten Datensatzes durch die Messstrahlung erfüllt werden, wird als Analyseergebnis die Speise, der Speisebestandteil, die Eigenschaft oder der Zustand des erkannten spektralen Fingerabdrucks bzw. des entsprechend zugeordneten Datensatzes ausgegeben. Lebensmittel, Inhaltsstoffe und Zustände können so anhand ihres spektralen Fingerabdrucks identifiziert werden. Insbesondere kann ein Analyseergebnis ein Bräunungsgrad, eine Alkoholkonzentration, ein Wassergehalt, ein Kakaoanteil in einer Schokolade, ein Glutenanteil in Mehl, ein Hauptnährstoffgehalt oder ein Energiegehalt sein. Das Analyseergebnis kann sowohl ein qualitatives Vorkommen eines Inhaltsstoffes als auch dessen quantitative Konzentration sein. Auch die Identifikation eines Speisebestandteils, z.B. "Apfel", ist möglich. Insbesondere kann eine besonders präzise Analyse durch einen Datenbankabgleich bzw. eine Berücksichtigung des Rezeptes oder der gekochten Speise erzielt werden, wobei diese Information beispielsweise durch eine Küchenmaschine oder ein Smartphone der Steuerungseinrichtung bereitgestellt werden kann.

In einer Ausführungsform wird ein Analyseergebnis dem Benutzer über eine Ausgabe des Speisezubereitungsgeräts insbesondere visuell, akustisch und/oder durch eine Übertragung auf ein externes Gerät wie z.B. Smartphone mitgeteilt. Insbesondere kann eine Küchenmaschine in Abhängigkeit von dem Analyseergebnis rezeptabhängig eine Empfehlung an den Benutzer ausgeben. Wenn beispielsweise wegen zu saurer Zutaten das geschmacklich empfohlene Zuckerniveau noch nicht erreicht ist, kann dem Benutzer ein Nachsüßen empfohlen werden.

In einer Ausführungsform sind das Spektrometer und/oder die Strahlquelle über eine drahtgebundene oder drahtlose Schnittstelle mit der Steuerungseinrichtung und/oder dem Speisezubereitungsgerät verbunden, um Messsignale und Steuersignale auszutauschen. Vorzugsweise sind die Steuerungseinrichtung mit einer Küchenmaschine und die Küchenmaschine mit einem Ofen für ein Austauschen von Daten verbunden.

Ein weiterer Aspekt der Offenbarung betrifft ein Verfahren zum Analysieren einer Speise, wobei durch ein Spektrometer die Speise oder ein Bestandteil der Speise bzw. ein Speisebestandteil vor, während und/oder nach einem Zubereiten der Speise analysiert wird, wobei die Zubereitung der Speise durch ein Speisenzubereitungsgerät mit einem Speisenzubereitungsraum sowie einem Heizelement zum Erhitzen einer Speise in dem Speisenzubereitungsraum und/oder einem Werkzeug zum Mischen und/oder Zerkleinern einer Speise in dem Speisenzubereitungsraum erfolgt. Insbesondere ist das Speisenzubereitungsgerät das Speisenzubereitungsgerät gemäß dem eingangs beschriebenen Aspekt der Erfindung, so dass die oben beschriebenen Merkmale und Ausführungsformen mit dem Verfahren kombiniert werden können.

Nachfolgend werden Ausführungsbeispiele der Erfindung sowie Beispiele, die das Verständnis der Erfindung erleichtern sollen, anhand von Figuren näher erläutert. Offenbarte Merkmale können beliebig mit den beanspruchten Gegenständen kombiniert werden. Die beanspruchten Schutzbereiche sind nicht auf die Ausführungsbeispiele beschränkt.

Es zeigen:
- Figur 1:: Schematische Darstellung eines Speisenzubereitungsgeräts mit Spektrometer;
- Figur 2:: Schematische Darstellung eines Speisenzubereitungsgeräts mit Spektrometer und flexibel einsetzbaren optischen Fasern gemäß der vorliegenden Erfindung;
- Figur 3:: Schematische Darstellung eines Speisenzubereitungsgeräts mit Spektrometer und optischen Fasern durch das Werkzeug zum Mischen und/oder Zerkleinern;
- Figur 4:: Schematische Darstellung eines Speisenzubereitungsgeräts mit Spektrometer und einem Analysefenster;
- Figur 5:: Schematische Darstellungen von Varianten der Anordnung eines Spektrometers und einer Strahlquelle in dem Speisenzubereitungsraum:
a) nebeneinander oberhalb einer Speise,
b) nebeneinander unterhalb einer Speise,
c) mit einem Heizelement als Strahlquelle,
d) mit einer Kamera (Spektrometer und Strahlquelle sind ausgeblendet) gemäß einer Ausführungsform der vorliegenden Erfindung,
e) mit einer Bewegungs- und Fokussiereinrichtung (Strahlquelle ausgeblendet),
f) mit der Strahlquelle oberhalb und dem Spektrometer unterhalb der Speise,
g) mit der Strahlquelle und dem Spektrometer an zwei gegenüberliegenden Endbereichen einer oberen Begrenzung des Speisenzubereitungsraums,
h) mit der Strahlquelle und dem Spektrometer an gegenüberliegenden Seitenwänden des Speisenzubereitungsraums.

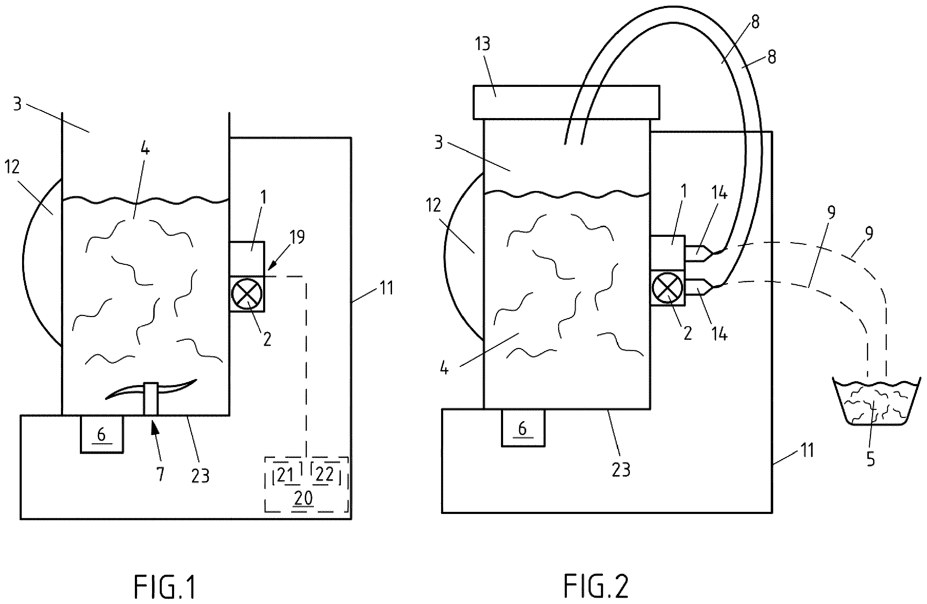

Die Figur 1 zeigt ein Speisenzubereitungsgerät mit einem Spektrometer 1 und einer Strahlquelle 2, die bevorzugt als eine Analyseeinheit 19 an dem Gefäß 12 mit dem Speisenzubereitungsraum 3 für eine Speise 4 oder dem Grundgerät 11 des Speisenzubereitungsgeräts verbunden oder verbindbar ist. Ein Werkzeug 7 und ein Heizelement 6 dienen der Zubereitung der Speise 4. Eine Steuerungseinrichtung 20 mit einer Prozessoreinheit 21 und einer Speichereinheit 22 ist mit dem Spektrometer 1 und/oder der Strahlquelle 2 verbunden. In einer Ausführungsform ist die Analyseeinheit 19 als ein tragbares Handgerät ausgestaltet, das der Benutzer für ein Analysieren auf eine zu untersuchende Speise richten kann. Bevorzugt ist die Analyseeinheit 19 jedoch fest mit dem Speisenzubereitungsraum 3, also dem Gefäß 12, einem Topf oder Ofenraum beispielsweise über ein optisches Analysefenster und/oder eine optische Faser wie nachfolgend beschrieben verbunden. Der Grund des Gefäßes 12 bildet die Auflage 23 für die Speise 4. Ist die Analyseeinheit als tragbares Handgerät ausgestaltet, dann kann die Analyseeinheit grundsätzlich so durch eine Halterung des Speisenzubereitungsgeräts gehalten werden, dass gewünschte Analysen durchgeführt werden können, ohne dafür die Analyseeinheit 19 manuell tragen zu müssen.

Die Figur 2 zeigt ein Speisenzubereitungsgerät, bei dem von der Strahlquelle 2 eine Anregungsstrahlung über eine optische Faser 8 in den Speisenzubereitungsraum 3 geleitet und eine Messstrahlung über eine zweite optische Faser 8 zum Spektrometer 1 zurückgeleitet werden, insbesondere jeweils durch einen Deckel 13. Eine optische Weiche 14, d.h. Y-Verteiler, ist vorgesehen, um eine zusätzliche optische Faser 9 jeweils an die Strahlquelle 2 und/oder das Spektrometer 1 anzukoppeln. Ein Speisebestandteil 5 außerhalb des Speisenzubereitungsraums 3 kann somit auch durch ein fest integriertes Spektrometer 1 analysiert werden.

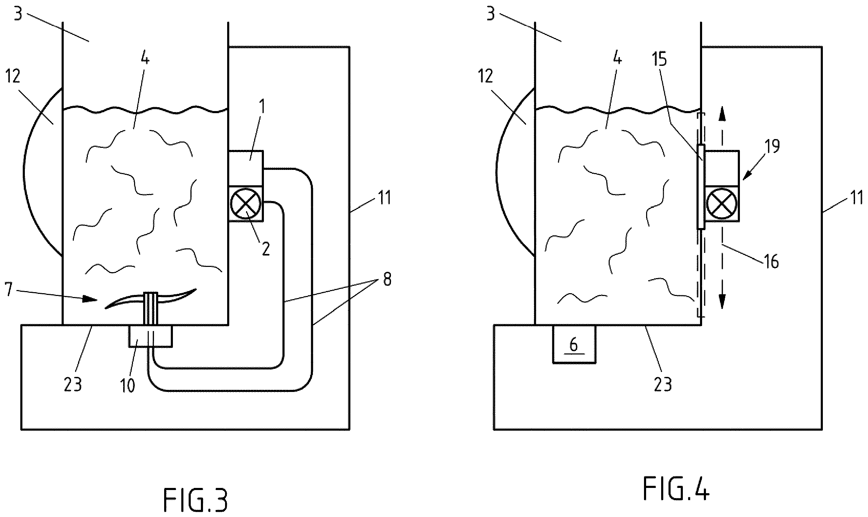

Die Figur 3 zeigt ein Speisenzubereitungsgerät, bei dem die optischen Fasern 8 der Strahlquelle 2 und/oder des Spektrometers 1 durch das Werkzeug 7 zum Mischen und/oder Zerkleinern bis zum Speisenzubereitungsraum 3 verlaufen, insbesondere entlang der Werkzeugachse. Bevorzugt ist eine Kupplungsschnittstelle 10 vorgesehen, um eine optische Übertragung der Anregungsstrahlung und/oder Messstrahlung ohne eine Bewegungskopplung zu erzielen, so dass sich das Werkzeug drehen kann, ohne dass sich die an die Strahlquelle 2 und/oder das Spektrometer 1 angekoppelten optischen Fasern 8 mitdrehen.

Die Figur 4 zeigt ein Speisenzubereitungsgerät mit einem Analysefenster 15 zum Übertragen der Anregungsstrahlung und/oder Messstrahlung zwischen dem Speisenzubereitungsraum 3 und der Strahlquelle 2 bzw. dem Spektrometer 1. Bevorzugt ist das Analysefenster 15 kratzfest, stoßfest, temperaturbeständig und/oder mit einem Wärmeausdehnungskoeffizienten ähnlich im Vergleich zur umgebenden Gefäßwand ausgestattet. In einer Ausführungsform erstreckt sich das Analysefenster 15 wie in Fig. 4 mit gestrichelter Linie dargestellt von einem unteren Randbereich oder Bereich des Gefäßes bis zu einem oberen Randbereich oder Bereich des Gefäßes. Die Analyseeinheit 19 kann durch eine Bewegungseinrichtung 16 entlang des Analysefensters 15 verfahren werden, um an verschiedenen Positionen die Speise 4 zu analysieren.

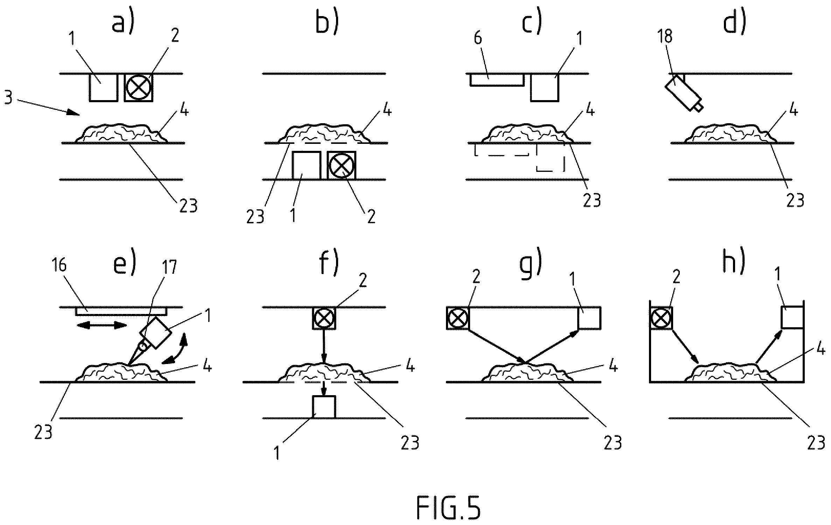

Die Figur 5 zeigt verschiedene Varianten der Anordnung eines Spektrometers 1 und einer Strahlquelle 2 in dem Speisenzubereitungsraum 3, der bevorzugt durch eine Küchenmaschine oder einen Ofen bereitgestellt ist. Das Spektrometer 1 und die Strahlquelle 2 sind in den Figuren 5a, 5b und 5c nahe nebeneinander oder aneinander angrenzend angeordnet. In den Fig. 5a und Fig. 5c wird die Speise 4 von oben analysiert. In Fig. 5b und einer Alternative der Fig. 5c (gestrichelt dargestellten) wird die Speise 4 von unten durch eine optisch durchlässige Auflage 23, z.B. eine transparente Scheibe oder ein Gitter, analysiert, was das Vorsehen eines definierten Abstand zur Speise erlaubt. In einer Ausführungsform, wie in Fig. 5c gezeigt, sind das Spektrometer 1, die Strahlquelle 2 und/oder das Heizelement unmittelbar mit der Auflage 23 verbunden. Bei dem Ausführungsbeispiel der Fig. 5c wird ein Heizelement 6 als Strahlquelle 2 genutzt. Das Heizelement 6 ist vorzugsweise ein Thermoelement einer Küchenmaschine oder ein Heizstab eines Ofens. Die Fig. 5d zeigt eine Kamera 18 zusätzlich zu einem Spektrometer 1 und einer Strahlquelle 2, die beide in Fig. 5d ausgeblendet sind. Bei den Figuren 5a bis 5d wird die Reflexionsstrahlung als die Messstrahlung für die Analyse genutzt, insbesondere mit einem spitzen Winkel zwischen der Anregungsstrahlung und der Messstrahlung. Die Fig. 5e illustriert eine Bewegungseinrichtung 16 zum translatorischen Bewegen und rotatorischen Neigen eines Spektrometers 1. Alternativ oder ergänzend kann die Bewegungseinrichtung 16 auch für die Strahlquelle 2 oder eine Analyseeinheit 19 eingesetzt werden. Eine Fokussiereinheit 17 ermöglicht ein besonders zielgerichtetes Erfassen von Messstrahlung von der Speise 4 und/oder Abgeben von Anregungsstrahlung auf die Speise 4. In Fig. 5f ist der Anordnung für die Analyse einer Transmissionsstrahlung als die Messstrahlung gezeigt, während in Fig. 5g und Fig. 5h Anordnungen für die Analyse einer Reflexionsstrahlung unter einem besonders großen Einfalls- und Ausfallswinkel von weniger als 60° zur Auflage 23 der Speise 4 gemessen illustriert sind.

## Patentansprüche

1. Speisenzubereitungsgerät mit einem Speisenzubereitungsraum (3) sowie einem Heizelement (6) zum Erhitzen einer Speise (4) in dem Speisenzubereitungsraum (3) und/oder einem Werkzeug (7) zum Mischen und/oder Zerkleinern einer Speise (4) in dem Speisenzubereitungsraum (3), wobei das Speisenzubereitungsgerät ein Spektrometer (1) und eine Strahlquelle (2) zum Analysieren einer Speise (4) umfasst, **dadurch gekennzeichnet, dass** eine optische Faser (8) vorgesehen ist, um eine Messstrahlung von einer Speise (4) zu dem Spektrometer (1) zu leiten und/oder eine Anregungsstrahlung von der Strahlquelle (2) zu einer Speise (4) zu leiten, wobei eine optische Weiche (14) vorgesehen und so beschaffen ist, dass die Anregungsstrahlung oder die Messstrahlung durch die optische Faser (8) und eine zusätzliche optische Faser (9) geleitet werden kann.

2. Speisenzubereitungsgerät nach dem vorhergehenden Anspruch, **dadurch gekennzeichnet, dass** eine Strahlquelle (2) für das Analysieren einer Speise (4) vorgesehen ist, die vorzugsweise Infrarot-Strahlung emittiert.

3. Speisenzubereitungsgerät nach einem der vorhergehenden Ansprüche, **dadurch gekennzeichnet, dass** die optische Faser (8) durch eine Öffnung in einem Gefäß (12) mit dem Speisenzubereitungsraum (3), in einem Deckel (13) für den Speisenzubereitungsraum (3) oder durch das Werkzeug (7) verläuft.

4. Speisenzubereitungsgerät nach dem vorhergehenden Anspruch, **dadurch gekennzeichnet, dass** eine optische Kupplungsschnittstelle (10) zum Anschließen der optischen Faser (8) an das Spektrometer (1), die Strahlquelle (2), das Gefäß (12), den Deckel (13) und/oder das Werkzeug (7) vorgesehen ist.

5. Speisenzubereitungsgerät nach einem der vorhergehenden Ansprüche, **dadurch gekennzeichnet, dass** ein Gefäß (12) mit dem Speisenzubereitungsraum (3) oder ein Deckel (13) für den Speisenzubereitungsraum (3) ein Analysefenster (15) aufweist.

6. Speisenzubereitungsgerät nach einem der vorhergehenden Ansprüche, **dadurch gekennzeichnet, dass** eine Bewegungseinrichtung (16) und/oder eine Fokussiereinrichtung (17) vorgesehen sind, so dass eine Anregungsstrahlung flexibel auf eine Speise (4) gerichtet werden kann und/oder eine Messstrahlung von einer Speise (4) flexibel erfasst werden kann.

7. Speisenzubereitungsgerät nach einem der vorhergehenden Ansprüche, **dadurch gekennzeichnet , dass** eine Kamera (18) vorgesehen ist, um eine Speise (4) digital abzubilden.

8. Speisenzubereitungsgerät nach einem der vorhergehenden Ansprüche, **dadurch gekennzeichnet, dass** das Spektrometer (1) und/oder die Strahlquelle (2) in einem Ofen oder einem Gefäß (12) mit dem Speisenzubereitungsraum (3) und/oder einem Grundgerät (11) zur Aufnahme des Gefäßes (12) integriert sind.

9. Speisenzubereitungsgerät nach einem der vorhergehenden Ansprüche, **dadurch gekennzeichnet, dass** das Spektrometer (1) und die Strahlquelle (2) als gemeinsame Analyseeinheit (19) vorgesehen sind und/oder an einen Ofen, an ein Gefäß (12) mit dem Speisenzubereitungsraum (3) und/oder an ein Grundgerät (11) zur Aufnahme des Gefäßes (12) ankoppelbar sind.

10. Verfahren zum Analysieren einer Speise (4), wobei durch ein Spektrometer (1) die Speise (4) oder ein Speisebestandteil (5) vor, während und/oder nach einem Zubereiten der Speise (4) analysiert wird, wobei die Zubereitung der Speise (4) durch ein Speisenzubereitungsgerät mit einem Speisenzubereitungsraum (3) sowie einem Heizelement (6) zum Erhitzen einer Speise (4) in dem Speisenzubereitungsraum (3) und/oder einem Werkzeug (7) zum Mischen und/oder Zerkleinern einer Speise (4) in dem Speisenzubereitungsraum (3) erfolgt, **dadurch gekennzeichnet, dass** durch eine optische Faser (8) eine Messstrahlung von einer Speise (4) zu dem Spektrometer (1) geleitet und/oder eine Anregungsstrahlung von einer Strahlquelle (2) zu einer Speise (4) geleitet wird, wobei eine optische Weiche (14) vorgesehen ist, so dass die Anregungsstrahlung oder die Messstrahlung durch die optische Faser (8) und eine zusätzliche optische Faser (9) geleitet werden kann.

11. Verfahren nach dem vorhergehenden Anspruch, **dadurch gekennzeichnet, dass** durch eine Kamera (18) eine Speise (4) digital abgebildet wird.

## Claims

1. Food preparation apparatus having a food preparation space (3) and a heating element (6) for heating a food (4) in the food preparation space (3) and/or a tool (7) for mixing and/or chopping a food (4) in the food preparation space (3), wherein the food preparation apparatus comprises a spectrometer (1) and a beam source (2) for analyzing a food (4), **characterized in that** an optical fiber (8) is provided for guiding a measurement radiation from a food (4) to the spectrometer (1) and/or for guiding an excitation radiation from the beam source (2) to a food (4), wherein an optical splitter (14) is provided and adapted such that the excitation radiation or the measurement radiation can be guided through the optical fiber (8) and an additional optical fiber (9).

2. Food preparation apparatus according to the preceding claim, **characterized in that** a radiation source (2) for analyzing a food (4) is provided, which preferably emits infrared radiation.

3. Food preparation apparatus according to one of the preceding claims, **characterized in that** the optical fiber (8) passes through an opening in a vessel (12) having the food preparation space (3), in a lid (13) for the food preparation space (3) or through the tool (7).

4. Food preparation apparatus according to the preceding claim, **characterized in that** an optical coupling interface (10) is provided for connecting the optical fiber (8) to the spectrometer (1), the beam source (2), the vessel (12), the lid (13) and/or the tool (7).

5. Food preparation apparatus according to one of the preceding claims, **characterized in that** a vessel (12) having the food preparation space (3) or a lid (13) for the food preparation space (3) has an analysis window (15).

6. Food preparation apparatus according to one of the preceding claims, **characterized in that** a moving device (16) and/or a focusing device (17) are provided so that an excitation radiation may be flexibly directed to a food (4) and/or a measurement radiation may be flexibly detected from a food (4).

7. Food preparation apparatus according to one of the preceding claims, **characterized in that** a camera (18) is provided for digitally imaging a food (4).

8. Food preparation apparatus according to one of the preceding claims, **characterized in that** the spectrometer (1) and/or the beam source (2) are integrated in an oven or a vessel (12) having the food preparation space (3) and/or a basic device (11) for receiving the vessel (12).

9. Food preparation apparatus according to one of the preceding claims, **characterized in that** the spectrometer (1) and the beam source (2) are provided as a common analysis unit (19) and/or can be coupled to an oven, to a vessel (12) with the food preparation space (3) and/or to a basic device (11) for receiving the vessel (12).

10. Method for analyzing a food (4), wherein the food (4) or a food component (5) is analyzed by a spectrometer (1) before, during and/or after a preparation of the food (4), wherein the preparation of the food (4) is carried out by a food preparation apparatus having a food preparation space (3) as well as a heating element (6) for heating a food (4) in the food preparation space (3) and/or a tool (7) for mixing and/or chopping a food (4) in the food preparation space (3), **characterized in that** measurement radiation is guided from a food (4) to the spectrometer (1) through an optical fiber (8) and/or excitation radiation is guided from a beam source (2) to a food (4), wherein an optical splitter (14) is provided so that the excitation radiation or the measurement radiation can be guided through the optical fiber (8) and an additional optical fiber (9).

11. Method according to the preceding claim, **characterized in that** a food (4) is digitally imaged by a camera (18).

## Revendications

1. Appareil de préparation d'aliments (1) comprenant un espace de préparation d'aliments (3) ainsi qu'un élément de chauffage (6) pour chauffer un aliment (4) dans l'espace de préparation d'aliments (3) et/ou un outil (7) pour mélanger et/ou broyer un aliment (4) dans l'espace de préparation d'aliments (3), l'appareil de préparation d'aliments (1) comprenant un spectromètre (1) et une source de rayonnement (2) pour analyser un aliment (4), **caractérisé en ce qu'**une fibre optique (8) est prévue pour guider un rayonnement de mesure à partir d'un aliment (4) vers le spectromètre (1) et/ou pour guider un rayonnement d'excitation de la source de rayonnement (2) vers un aliment (4), un séparateur optique (14) étant prévu et configuré, de sorte que le rayonnement d'excitation ou le rayonnement de mesure peut être guidé à travers la fibre optique (8) et une fibre optique supplémentaire (9).

2. Appareil de préparation d'aliments selon la revendication précédente, **caractérisé en ce qu'**une source de rayonnement (2) est prévue pour analyser un aliment (4), laquelle source de rayonnement émet de préférence des rayons infrarouges.

3. Appareil de préparation d'aliments selon l'une des revendications précédentes, **caractérisé en ce que** la fibre optique (8) s'étend à travers une ouverture dans un récipient (12) comprenant l'espace de préparation d'aliments (3) dans un couvercle (13) pour l'espace de préparation d'aliments (3) ou à travers l'outil (7).

4. Appareil de préparation d'aliments selon la revendication précédente, **caractérisé en ce qu'**une interface d'accouplement optique (10) est prévue pour raccorder la fibre optique (8) au spectromètre (1), à la source de rayonnement (2), au récipient (12), au couvercle (13) ou à l'outil (7).

5. Appareil de préparation d'aliments selon l'une des revendications précédentes, **caractérisé en ce qu'**un récipient (12) comprenant l'espace de préparation d'aliments (3) ou un couvercle (13) pour l'espace de préparation d'aliments (3) comprend une fenêtre d'analyse (15).

6. Appareil de préparation d'aliments selon l'une des revendications précédentes, **caractérisé en ce qu'**un dispositif de déplacement (16) ou un dispositif de focalisation (17) est prévu, de sorte qu'un rayonnement d'excitation peut être dirigé sur un aliment (4) de manière flexible et/ou un rayonnement de mesure peut être capturé de manière flexible par un aliment (4).

7. Appareil de préparation d'aliments selon l'une des revendications précédentes, **caractérisé en ce qu'**une caméra (18) est prévue pour reproduire un aliment (4) de manière numérique.

8. Appareil de préparation d'aliments selon l'une des revendications précédentes, **caractérisé en ce que** le spectromètre (1) et/ou la source de rayonnement (2) est/sont intégré(e)(s) dans un four ou dans un récipient (12) comprenant l'espace de préparation d'aliments (3) et/ou dans un dispositif de base (11) pour recevoir le récipient (12).

9. Appareil de préparation d'aliments selon l'une des revendications précédentes, **caractérisé en ce que** le spectromètre (1) et la source de rayonnement (2) sont prévus comme une unité d'analyse commune (19) et/ou ils sont susceptibles d'être couplés à un four, à un récipient (12) comprenant l'espace de préparation d'aliments (3) et/ou à un dispositif de base (11) pour recevoir le récipient (12).

10. Procédé d'analyse d'un aliment (4), dans lequel l'aliment (4) ou un composant d'aliment (5) est analysé avant, pendant et/ou après une préparation de l'aliment (4), l'aliment (4) étant préparé par moyen d'un appareil de préparation d'aliments comprenant un espace de préparation d'aliments (3) ainsi qu'un élément de chauffage (6) pour chauffer un aliment (4) dans l'espace de préparation d'aliments (3) et/ou un outil (7) pour mélanger et/ou broyer un aliment (4) dans l'espace de préparation d'aliments (3), **caractérisé en ce qu'**un rayonnement de mesure est guidé à partir d'un aliment (4) à travers une fibre optique (8) jusqu'au spectromètre (1) et/ou un rayonnement d'excitation est guidé à partir d'une source de rayonnement (2) à travers une fibre optique (8) vers un aliment (4), un séparateur optique (14) étant prévu, de sorte que le rayonnement d'excitation ou le rayonnement de mesure peut être guidé à travers la fibre optique (8) et une fibre optique supplémentaire (9).

11. Procédé selon la revendication précédente, **caractérisé en ce qu'**un aliment (4) est reproduit de manière numérique par moyen d'une caméra (18).
